(19) Europäisches Patentamt
European Patent Office
Office européen des brevets

(11) Numéro de publication: **0 021 939**
**B1**

(12) **FASCICULE DE BREVET EUROPEEN**

(45) Date de publication du fascicule du brevet: 12.01.83

(21) Numéro de dépôt: 80400830.8

(22) Date de dépôt: 10.06.80

(51) Int. Cl.³: C 07 D 239/46, C 07 D 239/48, A 01 N 43/54

(54) **Hydrazino (ou azido) alkylthio-5 pyrimidines, leurs procédés de préparation et leurs utilisations comme herbicides et fongicides.**

(30) Priorité: 20.06.79 FR 7915769

(43) Date de publication de la demande: 07.01.81 Bulletin 81/1

(45) Mention de la délivrance du brevet: 12.01.83 Bulletin 83/2

(84) Etats contractants désignés: BE CH DE FR GB IT LI LU NL SE

(56) Documents cités: EP - A - 0 000 681

(73) Titulaire: P C U K PRODUITS CHIMIQUES UGINE KUHLMANN
Service Propriété Industrielle Tour Manhattan
F-92087 Paris La Defense 2 Cédex 21 (FR)

(72) Inventeur: Balde, Daniel Henry
13, rue Charles Friedel
F-75020 Paris (FR)
Inventeur: Boutemy, Gérard Emile Marcel
19 Chemin des Résistants
Oncy-sur-Ecole F-91490 Milly la Forêt (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 021 939 B1

Hydrazino (ou azido) alkylthio-5-pyrimidines, leurs procédés de préparation et leurs utilisations comme herbicides et fongicides

La présente invention a pour objet de nouvelles alkylthio-5 pyrimidines portant un groupe hydrazino, hydrazino substitué ou azido, leurs procédés de préparation et leurs utilisations en tant que herbicides et fongicides.

Il est déjà connu, par les brevets français 2 031 422, 2 317 291, 2 137 933, 2 119 234, par l'article de MOSSINI, MAGGIALI, BRANCA Ateano Parmense, Acta Nat., 14 (1978), 119—126, et par la demande de brevet européen 78 400062.2 publiée sous le numéro 0 000 681, des dérivés de la pyrimidine herbicides, mais ces dérivés ne portent jamais simultanément un groupe alkylthio en position 5 et un groupe hydrazino, hydrazino substitué ou azido.

Les nouvelles alkylthio-5 pyrimidines selon l'invention peuvent être représentées par la formule générale:

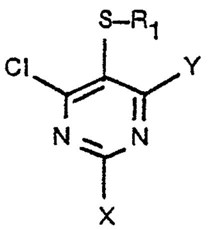

(I)

dans laquelle $R_1$ est un groupe alkyle ayant 1 à 5 atomes de carbone, l'un des substituants X et Y est un atome de chlore ou un groupe

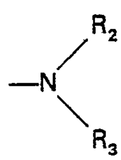

dans lequel $R_2$ est un atome d'hydrogène ou un groupe alkyle ayant 1 à 5 atomes de carbone et $R_3$ est un atome d'hydrogène ou un groupe alkyle ayant 1 à 5 atomes de carbone ou benzyle, et l'autre est un groupe azido, hydrazino, ou un groupe hydrazino substitué répondant à l'une des formules

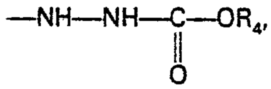 , 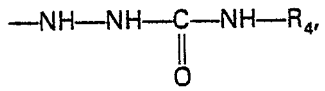 , 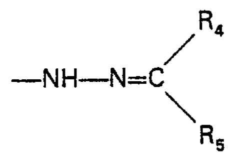

dans lesquelles $R_4$ et $R_5$ sont des groupes alkyle ayant 1 à 5 atomes de carbone, ou bien les substituants X et Y sont identiques et représentent alors des groupes azido, hydrazino, ou hydrazino substitués de formule

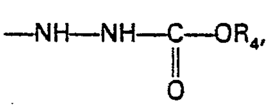 , 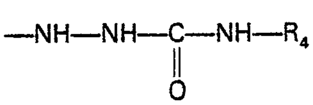 ou 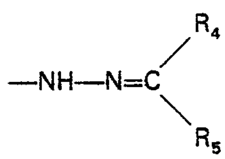

formules dans lesquelles $R_4$ et $R_5$ ont les significations précitées.

Bien que la présente invention concerne les composés de formule (I) dans leur ensemble, elle a plus particulièrement pour objet ceux de ces composés pour lesquels $R_1$ est un groupe méthyle, l'un des substituants X et Y est un atome de chlore ou un groupe amino, méthylamino et l'autre est un groupe azido, hydrazino,

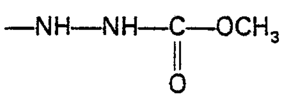 ou 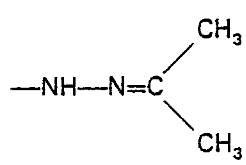 ,

ou bien les deux substituants X et Y sont des groupes hydrazino.

Les composés de formule (I) dans lesquels l'un au moins des substituants X et Y est un groupe azido ou un groupe hydrazino substitué de formule

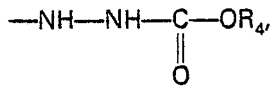

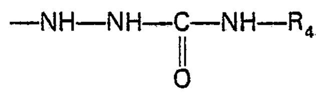

ou

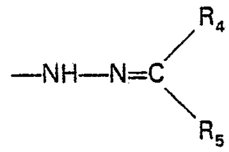

peuvent être préparés par action, sur le composé de formule (I) correspondant dans lequel ledit substituant ou lesdits substituants sont des groupes hydrazino, de respectivement l'acide nitreux, un chloroformiate de formule

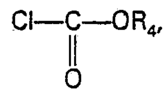

un isocyanate de formule $R_4$—N=C=O, une cétone de formule

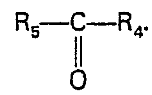

Pour préparer les composés de formule (I) ayant un ou deux substituants azido par action de l'acide nitreux sur les composés correspondants de formule (I) ayant un ou deux substituants hydrazino, on peut par exemple chauffer ces derniers à une température de 10°C à 100°C en milieu acide en présence d'un nitrite d'un métal alcalin ou alcalino-terreux. L'acide nitreux nécessaire à la réaction est produit par l'action de l'acide sur le nitrite. Comme milieu acide on peut citer en particulier les milieux mixtes eau + acide acétique.

La réaction des composés de formule (I) ayant un ou deux substituants hydrazino avec le chloroformiate de formule

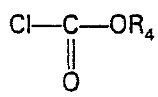

peut être effectuée par exemple à une température de 0°C à 20°C, en présence d'un solvant et d'une base pour fixer l'acide chlorhydrique libéré. Il est commode d'opérer au sein de la pyridine qui sert à la fois de solvant et de base.

La réaction des composés de formule (I) ayant un ou deux substituants hydrazino avec l'isocyanate $R_4$—N=C=O peut être effectuée dans un solvant inerte vis-à-vis de l'isocyanate, par exemple le tétrahydrofuranne, à une température allant de 20°C jusqu'à la température d'ébullition du solvant.

La réaction des composés de formule (I) ayant un ou deux substituants hydrazino avec la cétone

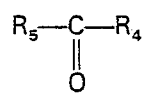

peut être effectuée par exemple en chauffant au reflux lesdits composés au sein de la cétone.

Les composés de formule (I) dans lesquels l'un des substituants X et Y est un atome de chlore ou un groupe

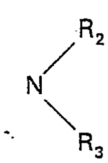

et l'autre est un groupe hydrazino peuvent être préparés par condensation d'une mole d'hydrazine avec une mole d'une trichloro-2,4,6 alkylthio-5 pyrimidine de formule (II) et condensation, mole à mole, de la dichloro-4,6 (ou -2,6) hydrazino-2 (ou -4) alkylthio-5 pyrimidine de formule (III) ou (III bis) ainsi d'ébullition du solvant utilisé. Elle est effectuée avantageusement à une température de 5°C à 25°C.

(1) 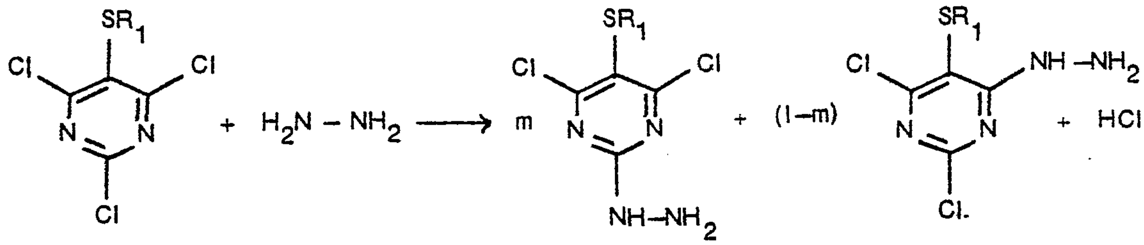

(II) (III) (III bis)

(2) 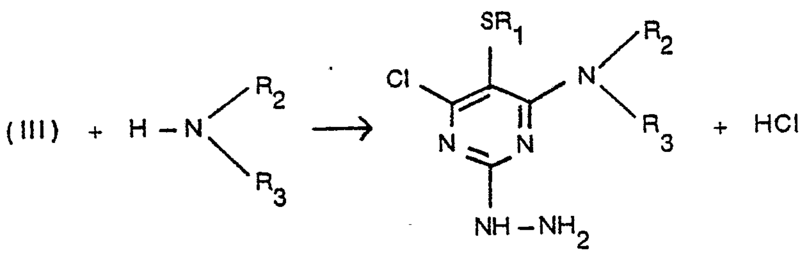

(IV) (I)

(2 bis) 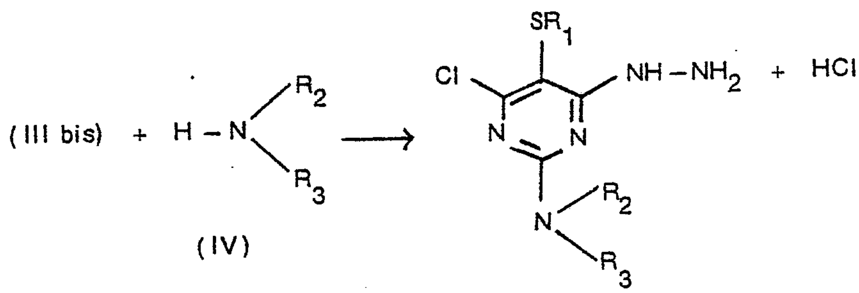

(IV)

(I bis)

Dans les formules (II) à (IV), $R_1$, $R_2$ et $R_3$ ont les mêmes significations que dans la formule (I). m est un nombre supérieur à 0 et inférieur à 1.

La trichloro-2,4,6 méthylthio-5 pyrimidine, produit de formule (II) pour lequel $R_1 = CH_3$, est un composé connu, qui a été décrit par exemple dans le brevet français 1 549 494. Les autres composés de formule (II) peuvent être préparés par le procédé général décrit dans ce brevet.

On peut aussi soumettre le mélange des composés (III) et (III bis) obtenu dans la réaction (1) à la deuxième étape du procédé (réaction avec le composé IV). On obtient alors un mélange des deux composés isomères de position (I) et (I) bis, mélange qui peut être employé tel quel dans les applications herbicides et fongicides.

Les réactions de condensation (1), (2) et (2 bis) peuvent être effectuées en milieu aqueux, en milieu solvant organique ou encore en milieu mixte eau + solvant organique. Comme solvants organiques utilisables on peut citer en particulier le benzène et le toluène.

Les réactions de condensation (1), (2) et (2) bis sont effectuées en présence d'un agent basique susceptible de fixer l'acide chlorhydrique formé dans la réaction. Cet agent basique peut être l'hydrazine elle-même ou le composé de formule (IV) lui-même dans le cas respectivement des réactions (1) et (2) ou (2 bis).

La quantité d'hydrazine mise en jeu pour effectuer la réaction (1) est la quantité stoechiométrique, c'est-à-dire 1 mole par mole de composé (II) dans le cas où l'agent basique utilisé pour fixer HCl est autre que l'hydrazine elle-même et 2 moles par mole de composé (II) dans le cas où l'agent basique utilisé pour fixer HCl est l'hydrazine elle-même.

La réaction (1) peut être réalisée à une température allant de 0°C jusqu'à la température d'ébullition du solvent utilisé. Elle est effectuée avantageusement à une température de 5°C à 25°C. Les réactions (2) et (2) bis ne peuvent être réalisées à des températures aussi basses que celles

utilisables pour la réaction (1). Elles sont effectuées en général entre 100 et 150°C, éventuellement sous une pression supérieure à la pression atmosphérique si la nature du solvant utilisé l'exige.

Les composés de formule (I) dans lesquels l'un des substituants X et Y est un groupe

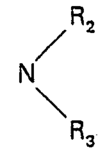

et l'autre est un groupe hydrazino peuvent aussi être préparés par condensation, mole à mole, de l'hydrazine avec un composé de formule (V) ou (V bis), suivant le schéma réactionnel:

(3)

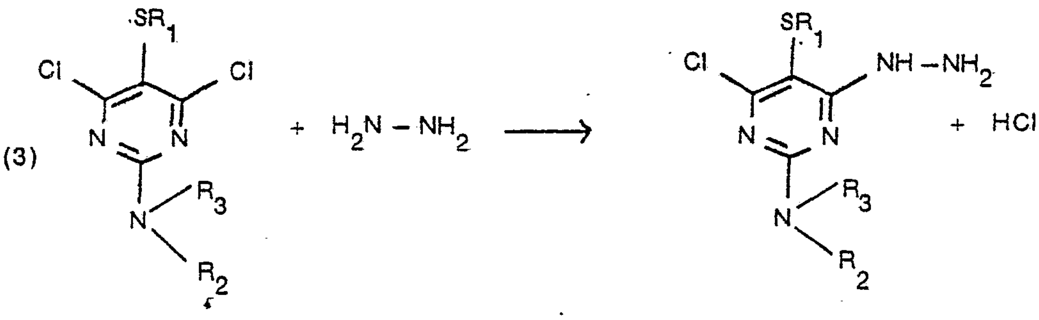

(V) (I ter)

(3 bis)

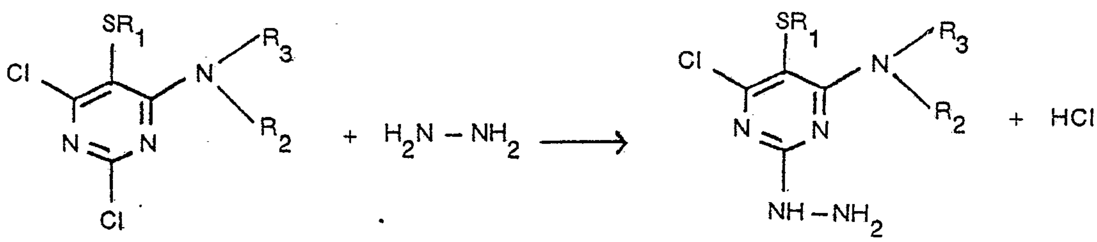

(V bis) (I quart)

On peut également soumettre le mélange des composés (V) et (V bis) résultant de la condensation mole à mole de la trichloro-2,4,6 alkylthio-5 pyrimidine de formule (II) avec un composé de formule

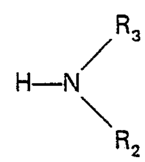

à la réaction de condensation avec l'hydrazine. On obtient alors un mélange des deux composés isomères de position (I ter) et (I quart), mélange qui peut être employé tel quel dans les applications herbicides et fongicides.

La préparation des composés de formules (V) et (V) bis et des mélanges de composés isomères correspondants a été décrite en particulier dans les brevets français 2 119 234, 2 173 746, 2 257 294 et 2 281 117.

Les réactions de condensation (3) et (3 bis) peuvent être effectuées en milieu aqueux, en milieu solvant organique ou encore en milieu mixte eau + solvant organique. Elles sont effectuées à une température allant de 25°C à la température d'ébullition du solvant utilisé et en présence d'un agent basique susceptible de fixer l'acide chlorhydrique formé dans la réaction.

Enfin les composés de formule (I) pour lesquels X et Y sont tous les deux des groupes hydrazino peuvent être préparés, en une seule étape, par condensation de l'hydrazine avec une trichloro-2,4,6 alkylthio-5 pyrimidine de formule (II) suivant le schéma réactionnel:

(4) 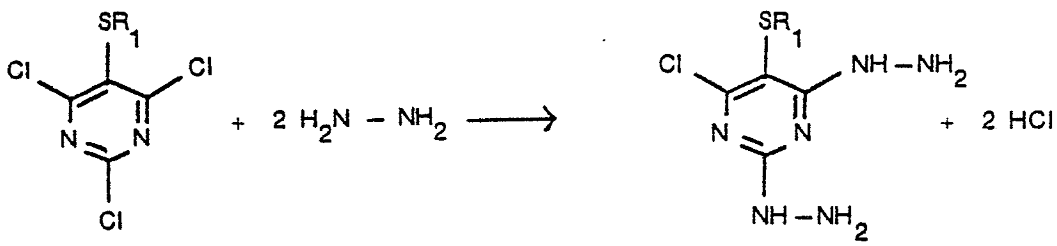

(II)

La réaction (4) est effectuée dans les mêmes conditions que la réaction (1), sauf en ce qui concerne la quantité d'hydrazine mise en jeu. Pour effectuer la réaction (4) on met en jeu une quantité d'hydrazine supérieure à la quantité stoechiométrique.

Les composés de formule (I) sont des herbicides actifs dans les traitements de pré- et post-levée. Ils ont la propriété de détruire un grand nombre de plantes indésirables appartement en particulier à la classe des dicotylédones et ce souvent à des doses inférieures ou égales à 2 500 g/ha. En outre, aux doses auxquelles ils sont actifs vis-à-vis des plantes indésirables, les composés de formule (I) présentent fréquemment une phytotoxicité nulle ou très faible vis-à-vis de céréales telles que le blé et le maïs.

Pour leur mise en oeuvre les composés herbicides selon l'invention peuvent être incorporés, conjointement avec d'autres herbicides ou séparément, dans des formulations qui contiennent, outre la matière active, les additifs inertes habituellement utilisés en agriculture pour faciliter la conservation, la mise en suspension aqueuse, l'adhérence sur le feuillage et la résistance aux agents atmosphériques et aux dégradations biologiques (d'où une persistance plus grande de l'action), tels que diluants solides (talc, silice, kieselguhr, argile, etc...) ou liquides (huiles minérales, eau, solvants organiques comme par exemple des cétones, des alcools, des hydrocarbures ou leurs dérives chlorés), adjuvants, tensio-actifs, antioxydants et stabilisants. De telles formulations peuvent se présenter sous la forme de poudres mouillables, solutions émulsifiables dans l'eau, suspensions, granulés ou toute autre forme en usage dans le domaine des herbicides.

Dans les formulations contenant seulement des composés herbicides selon l'invention et des additifs inertes, la teneur en composés de formule (I) (matière active) peut varier de 1% à 95% en poids. Dans les formulations contenant des composés herbicides selon l'invention, d'autres herbicides et des additifs inertes, la teneur en composés selon l'invention peut varier de 1% à 80% en poids, celle en herbicides autres de 80% à 1% en poids, le complément à 100% étant constitué par les additifs inertes.

En outre certains des composés de formule (I) possèdent des propriétés fongicides.

Les exemples suivants illustrent l'invention sans la limiter. Les données relatives aux spectres de résonance magnétique nucléaire (spectres R.M.N.) figurant dans ces exemples ont été obtenues en mettant les produits en solution dans le diméthylsulfoxyde deutéré.

## Exemple 1

Préparation d'un mélange des deux isomères dichloro-4,6 hydrazino-2 méthylthio-5 pyrimidine et dichloro-2,6 hydrazino-4 méthylthio-5 pyrimidine.

Dans un réacteur maintenu à 5°C et contenant 120 g de toluène et 46 g de trichloro-2,4,6 méthylthio-5 pyrimidine on introduit 65 g d'une solution aqueuse à 32,3% d'hydrate d'hydrazine. On laisse réagir 3 heures à 5°C, puis une nuit à 20°C. On filtre le précipité obtenu, le lave et le sèche. On obtient ainsi 40,5 g (ce qui correspond à un rendement de 90%) d'un produit constitué par un mélange de dichloro-4,6 hydrazino-2 méthylthio-5 pyrimidine et dichloro-2,6 hydrazino-4 méthylthio-5 pyrimidine.

Point de fusion du mélange: 150—152°C (avec décomposition).

Spectre R.M.N. du produit obtenu:

Les déplacements chimiques $\delta$ sont les suivants:

$SCH_3$ : $\delta = 2,2$ ppm

protons mobiles: $\delta = 6,56$ ppm

## Exemple 2

Préparation d'un mélange des deux isomères amino-2 chloro-6 hydrazino-4 méthylthio-5 pyrimidine et amino-4 chloro-6 hydrazino-2 méthylthio-5 pyrimidine.

*1ère étape*

Dans 500 ml d'eau contenant 1,2 g de PLURONIC L 92 (agent tensio-actif non ionique constitué par un copolymère d'oxyde d'éthylène et d'oxyde de propyléne) on disperse par agitation 46 g de trichloro-2,4,6 méthylthio-5 pyrimidine finement broyée. Puis on introduit en 10 minutes 170 g d'une

solution aqueuse d'ammoniac à 20%, en maintenant la température à 5°C. On laisse ensuite une nuit à la température ambiante, puis on filtre le précipité formé et le lave à l'eau. On recueille ainsi 42 g d'un produit qui est un mélange des deux composés isomères dichloro-4,6 amino-2 méthylthio-5 pyrimidine et dichloro-2,6 amino-4 méthylthio-5 pyrimidine (mélange dénommé D par la suite).

*2ème étape*

Dans un réacteur contenant 100 ml de toluène et 7 g du mélange D on introduit 155 g d'une solution aqueuse à 32,3% d'hydrate d'hydrazine. On laisse réagir 7 heures à 25°C, puis on filtre le précipité obtenu, le lave à l'eau et le sèche. On obtient ainsi 6 g (ce qui correspond à un rendement de 88,4%) d'un produit constitué par un mélange d'amino-2 chloro-6 hydrazino-4 méthylthio-5 pyrimidine et d'amino-4 chloro-6 hydrazino-2 méthylthio-5 pyrimidine. Ce mélange fond à 170°C (avec décomposition).

Spectre R.M.N. du produit obtenu:

$SCH_3$ : $\delta = 2,11$ ppm
$NH_2$ : $\delta = 4,2$ ppm
$NH—NH_2$ : $\delta = 6,7;\ 6,96;\ 8,2$ ppm

Exemple 3

Préparation de l'amino-2 chloro-6 hydrazino-4 méthylthio-5 pyrimidine et de l'amino-4 chloro-6 hydrazino-2 méthylthio-5 pyrimidine.

40 g du mélange D obtenu dans la lère étape de l'exemple 2 sont dissous dans 700 ml d'acide chlorhydrique concentré. A la solution obtenue on ajoute 200 ml d'eau. Il se forme un précipité a que l'on sépare par filtration. On ajoute au filtrat 120 ml d'eau. Il se forme un nouveau précipité b que l'on sépare par filtration. On ajoute enfin au filtrat 260 ml d'eau puis 200 ml d'une solution

$$\frac{N}{10}$$

d'hydroxyde de sodium. Il se forme un précipité c que l'on sépare par filtration.

Le précipité a (poids 6 g) est constitué essentiellement du composé dichloro-4,6 amino-2 méthylthio-5 pyrimidine. Le précipité c (poids 17,2 g) est constitué du composé dichloro-2,6 amino-4 méthylthio-5 pyrimidine.

En remplaçant dans la deuxième étape de l'exemple 2 les 7 g de mélange D par respectivement 7 g du précipité a et 7 g du précipité c, on obtient l'amino-2 chloro-6 hydrazino-4 méthylthio-5 pyrimidine et l'amino-4 chloro-6 hydrazino-2 méthylthio-5 pyrimidine.

Exemple 4

Préparation de la méthylamino-2 chloro-6 hydrazino-4 méthylthio-5 pyrimidine.

Dans un réacteur, muni d'un agitateur, on introduit 500 ml de benzène, 500 ml d'eau, 44,8 g de dichloro-4,6 méthylamino-2 méthylthio-5 pyrimidine et 30 g d'hydrate d'hydrazine. On chauffe au reflux pendant 3 heures sous bonne agitation, puis on refroidit et filtre le précipité obtenu. Le précipité est ensuite lavé à l'eau, puis au méthanol et est séché. On obtient ainsi 23 g (ce qui correspond à un rendement de 52,3%) de méthylamino-2 chloro-6 hydrazino-4 méthylthio-5 pyrimidine qui fond à 176°C (avec décomposition).

*Analyse élémentaire:*

| | %C | %H | %N |
|---|---|---|---|
| Calculé pour $C_6H_{10}N_5SCl$ | 32,8 | 4,55 | 31,9 |
| Trouvé | 33,1 | 4,59 | 31,5 |

Exemple 5

Préparation d'un mélange des deux isomères amino-2 chloro-6 (méthoxycarbonyl-2) hydrazino-4 méthylthio-5 pyrimidine et amino-4 chloro-6 (méthoxycarbonyl-2) hydrazino-2 méthylthio-5 pyrimidine.

Dans un réacteur contenant 160 ml de pyridine et 20,55 g du produit obtenu à la 2ème étape de l'exemple 2 on introduit 10,3 g de chloroformiate de méthyle, en maintenant la température à 0°C. On laisse réagir 4 heures à 0°C, puis 15 heures à 20°C. On élimine ensuite la pyridine par distillation sous vide. Le résidu obtenu est lavé à l'eau et séché. On obtient ainsi 8 g (ce qui correspond à un rendement de 30,3%) d'un produit constitué par un mélange d'amino-2 chloro-6 (méthoxycarbonyl-2) hydrazino-4 méthylthio-5 pyrimidine et d'amino-4 chloro-6 (méthoxycarbonyl-2) hydrazino-2 méthylthio-5 pyrimidine. Ce mélange fond à 208°C.

Spectre infra-rouge du produit obtenu:

bande d'absorption de CO à 1 700 $cm^{-1}$

Spectre R.M.N. du produit obtenu:

$SCH_3$ : $\delta = 2,1$ ppm
$OCH_3$ : $\delta = 3,56$ ppm
protons mobiles: $\delta = 7,1$ et 9 ppm

Exemple 6

Préparation de la méthylamino-2 chloro-6 azido-4 méthylthio-5 pyrimidine.

Dans un réacteur contenant 85 ml d'eau, 430 ml d'acide acétique et 30 g de méthylamino-2 chloro-6 hydrazino-4 méthylthio-5 pyrimidine (préparée comme indiqué à l'exemple 4) on introduit lentement, à la température ambiante (20°C à 25°C), 17 g de nitrite de sodium. On chauffe ensuite le mélange à 50°C et maintient cette température pendant 4 heures. Puis on amène à sec, lave à l'eau le résidu obtenu et le sèche. On obtient ainsi 10 g d'un produit brut. Pour purifier ce produit, on le dissout dans un mélange de 150 ml d'acide chlorhydrique concentré et 50 ml d'eau, puis fait reprécipiter par addition de 300 ml d'eau. On obtient ainsi 4,2 g de méthylamino-2 chloro-6 azido-4 méthylthio-5 pyrimidine, qui fond à 142—144°C.

*Analyse élémentaire:*

| | %C | %H | %N |
|---|---|---|---|
| Trouvé | 31,23 | 3,03 | 36,44 |

Exemple 7

Préparation d'un mélange des deux isomères amino-2 chloro-6 (méthyl-4 semicarbazido)-4 méthylthio-5 pyrimidine et amino-4 chloro-6 (méthyl-4 semicarbazido)-2 méthylthio-5 pyrimidine.

Dans un réacteur muni d'un agitateur on introduit 35 ml de tétrahydrofuranne, 4,1 g du produit obtenu à la 2ème étape de l'exemple 2 et 1,26 g d'isocyanate de méthyle. On laisse réagir à 20°C pendant 15 heures, puis à l'ébullition pendant 2 heures. On filtre après refroidissement. On obtient ainsi 4,6 g (ce qui correspond à un rendement de 87,5%) d'un produit constitué par un mélange d'amino-2 chloro-6 (méthyl-4 semicarbazido)-4 méthylthio-5 pyrimidine et d'amino-4 chloro-6 (méthyl-4 semicarbazido)-2 méthylthio-5 pyrimidine, qui fond à 217°C.

Spectre infra-rouge du produit obtenu:

bande d'absorption de CO à 1630 $cm^{-1}$

Spectre R.M.N. du produit obtenu:

$SCH_3$ : $\delta = 2,13$ ppm
N—$CH_3$ : $\delta = 2,52$ ppm

Exemple 8

Préparation de la bis(hydrazino)-2,4 chloro-6 méthylthio-5 pyrimidine.

Dans un réacteur contenant 100 ml de toluène et 7,5 g de trichloro-2,4,6 méthylthio-5 pyrimidine on introduit 155 g d'une solution aqueuse à 32,3% d'hydrate d'hydrazine. On laisse réagir 24 heures à 20°C puis on filtre. Le précipité obtenu est lavé à l'eau et séché. On obtient ainsi 5,9 g de bis(hydrazino)-2,4 chloro-6 méthylthio-5 pyrimidine (ce qui correspond à un rendement de 81,9%)

Exemple 9

Préparation de la benzylamino-2 chloro-6 hydrazino-4 méthylthio-5 pyrimidine.

Dans un réacteur contenant 23 g de trichloro-2,4,6 méthylthio-5 pyrimidine, 74 g de méthyléthylcétone et 65 g de glace, on coule en 30 minutes 10, 7 g de benzylamine. Puis on ajoute, à la température de 20°C, en l'espace d'une heure, une solution de 4 g d'hydroxyde de sodium dans 15 ml d'eau. On agite pendant 6 heures, puis on filtre et lave le précipité obtenu avec de la méthyléthylcétone. On obtient ainsi 10 g de benzylamino-2 dichloro-4,6 méthylthio-5 pyrimidine.

On fait réagir pendant 3 heures à l'ébullition, dans un milieu constitué de 85 ml de benzène et 85 ml d'eau, les 10 g de benzylamino-2 dichloro-4,6 méthylthio-5 pyrimidine obtenus précédemment avec 5 g d'hydrate d'hydrazine. On filtre et lave le précipité obtenu à l'eau, puis au méthanol. On obtient ainsi 4,8 g (ce qui correspond à un rendement de 48,7%) de benzylamino-2 chloro-6 hydrazino-4 méthylthio-5 pyrimidine.

Exemple 10

Préparation de la méthylamino-2 chloro-6 (isopropylidène-2 hydrazino)-4 méthylthio-5 pyrimidine.

On chauffe au reflux pendant 2 heures 13,2 g de méthylamino-2 chloro-6 hydrazino-4 méthylthio-5 pyrimidine (préparée comme indiqué à l'exemple 4) avec 200 ml d'acétone pure. Après refroidissement, on filtre le précipité formé. On obtient ainsi 8 g de méthylamino-2 chloro-6 (isopropylidène-2 hydrazino)-4 méthylthio-5 pyrimidine, qui fond à 190°C.

*Analyse élémentaire du produit obtenu:*

| | %C | %H | %N |
|---|---|---|---|
| Calculé pour $C_9H_{14}N_5SCl$ | 41,62 | 5,39 | 26,97 |
| Trouvé | 41,85 | 5,29 | 26,82 |

La structure du produit obtenu est confirmé par ses spectres infra-rouge et R.M.N.

Exemple 11

Préparation d'un mélange d'amino-2 chloro-6 azido-4 méthylthio-5 pyrimidine et d'amino-4 chloro-6 azido-2 méthylthio-5 pyrimidine.

Dans un réacteur de 1 litre contenant 90 ml d'eau, 450 ml d'acide acétique et 30,8 g du mélange d'amino-2 chloro-6 hydrazino-4 méthylthio-5 pyrimidine et d'amino-4 chloro-6 hydrazino-2 méthylthio-5 pyrimidine obtenu à la 2ème étape de l'exemple 2, on introduit lentement, à la température ambiante (20°C à 25°C), 18,6 g de nitrite de sodium. On chauffe ensuite le mélange à 50°C et maintient cette température pendant 4 heures. Puis on amène à sec, lave à l'eau le résidu obtenu et le sèche. On obtient ainsi 16 g d'un mélange d'isomères contenant environ 30% d'amino-2 azido-4 chloro-6 méthylthio-5 pyrimidine et 70% d'amino-4 azido-2 chloro-6 méthylthio-5 pyrimidine. Ce mélange fond à 117°C.

Spectre infra-rouge du produit obtenu:

bande d'absorption de $N_3$ à 2 140 $cm^{-1}$

Spectre R.M.N. du produit obtenu:

$SCH_3$ : $\delta = 2,15$ ppm et 2,45 ppm

Exemple 12

Activité herbicide des composés

Les composés selon l'invention sont formulés sous forme de suspensions aqueuses contenant 5% d'un tensio-actif dénommé "TWEEN 20".

Les quantités de suspensions appliquées équivalent à 1 000 1/ha, et les dilutions réalisées sont calculées de façon à apporter les quantités de matière active suivantes:

$$\begin{cases} D_1 = 2,5 \text{ kg/ha} \\ D_2 = 10 \text{ kg/ha} \end{cases}$$

Les suspensions sont appliquées au jour J. par pulvérisation, soit sur plantes âgées de 10 jours, ce qui permet d'étudier l'action de poste-levée des produits, soit sur semences déposées à la surface du sol, ce qui permet d'étudier l'action de pré-levée. Ces semences sont recouvertes de 2 cm de terre juste après l'application.

Les plantes et graines sont disposées dans des conteneurs en plastique de 18 x 12 x 5 cm remplis d'une terre standard composée de 3 parties de sable, 1 partie de terreau et 1 partie d'argile. Après traitement, les conteneurs sont disposés sur une tablette à irrigation automatique dans une serre maintenue à 22°C et à un taux d'hygrométrie de 70%.

Les plantes soumises aux essais sont le blé TRITICUM SP, le haricot PHASEOLUS SP, la betterave BETA SP, la moutarde SINAPIS SP, le pissenlit TARAXACUM SP et le maïs ZEA SP.

On relève les résultats 14 jours après le traitement dans le cas dès traitements de post-levée et 21 jours après le traitement dans le cas des traitements de pré-levée.

Les résultats relatifs aux composés des exemples 1, 2, 5, 8, 10 et 11 sont rassemblés dans le tableau n° I.

Dans ce tableau l'efficacité herbicide des composés selon l'invention vis-à-vis des plantes testées est exprimée par un chiffre qui représente le pourcentage de destruction des plantes dans les lots traités. Ce pourcentage est évalué en prenant comme référence les plantes de lots témoins non traités. Le chiffre 0 indique donc que l'état des plantes est le même dans les lots traités et dans les lots témoins, le chiffre 100 que les plantes sont entièrement détruites dans les lots traités, ce qui correspond à l'efficacité maximum.

Exemple 13

Activité herbicide des composés

On conduit les essais comme à l'exemple 12 avec les doses suivantes du composé de l'exemple 6 (méthylamino-2 chloro-6 azido-4 méthylthio-5 pyrimidine):

$D_1 = 0,312$ kg/ha

$D_2 = 0,625$ kg/ha

$D_3 = 1,25$ kg/ha

$D_4 = 2,5$ kg/ha

$D_5 = 5$ kg/ha

Les traitements sont uniquement des traitements de post-levée. Les notations sont effectuées 7, 14 et 21 jours après le traitement Les résultats obtenus sont rassemblés dans le tableau II. Dans ce tableau figurent aussi les résultats relatifs à un herbicide de référence (chlortoluron).

TABLEAU I

EFFICACITE HERBICIDE EN POURCENTAGE DE DESTRUCTION

(évaluation au jour J + 14 pour les traitements de post-levée et au jour J + 21 pour les traitements de pré-levée)

| | PRODUIT | DOSE (en kg/ha) | PLANTES | | | | | |
|---|---|---|---|---|---|---|---|---|
| | | | Blé | Haricot | Betterave | Moutarde | Pissenlit | Maïs |
| POST-LEVEE | Exemple 5 | 2,5 | 10 | 0 | 100 | 80 | 100 | 15 |
| | idem | 10 | 15 | 80 | 100 | 100 | 100 | 40 |
| | Exemple 8 | 10 | 0 | 0 | 100 | 100 | 0 | 0 |
| | Exemple 10 | 2,5 | 0 | 25 | 100 | 80 | 0 | 0 |
| | idem | 10 | 0 | 80 | 100 | 100 | – | 0 |
| | Exemple 1 | 10 | 0 | 0 | 0 | 80 | 100 | 0 |
| | Exemple 2 | 2,5 | 0 | 50 | 50 | 50 | 100 | 0 |
| | idem | 10 | 50 | 65 | 100 | 100 | 100 | 0 |
| | Exemple 11 | 2,5 | 15 | 100 | 100 | 100 | 100 | 5 |
| | idem | 10 | 40 | 100 | 100 | 100 | 100 | 5 |
| PRE-LEVEE | Exemple 11 | 2,5 | 0 | 0 | 100 | 30 | 100 | 0 |
| | idem | 10 | 0 | 30 | 100 | 100 | 100 | 0 |

TABLEAU II

APPLICATIONS DE POST-LEVEE
EFFICACITE HERBICIDE EN POURCENTAGE DE DESTRUCTION

| PRODUIT | DOSES | PLANTES | | | | | | | | | | | | | | | | | |
|---|---|---|---|---|---|---|---|---|---|---|---|---|---|---|---|---|---|---|---|
| | | Blé | | | Haricot | | | Betterave | | | Moutarde | | | Pissenlit | | | Maïs | | |
| | | J + 7 | J + 14 | J + 21 | J + 7 | J + 14 | J + 21 | J + 7 | J + 14 | J + 21 | J + 7 | J + 14 | J + 21 | J + 7 | J + 14 | J + 21 | J + 7 | J + 14 | J + 21 |
| Exemple 6 | 0,312 kg/ha | 0 | 5 | 0 | 40 | 28 | 8 | 100 | 83 | 93 | 40 | 74 | 75 | 49 | 80 | 78 | 0 | 0 | 0 |
| idem | 0,625 kg/ha | 0 | 10 | 20 | 55 | 48 | 26 | 100 | 100 | 100 | 88 | 100 | 100 | 94 | 100 | 100 | 0 | 0 | 0 |
| idem | 1,25 kg/ha | 8 | 18 | 20 | 68 | 76 | 63 | 100 | 100 | 100 | 74 | 100 | 100 | 100 | 100 | 100 | 0 | 3 | 0 |
| idem | 2,5 kg/ha | 13 | 39 | 20 | 74 | 80 | 99 | 100 | 100 | 100 | 98 | 100 | 100 | 100 | 100 | 100 | 75 | 10 | 0 |
| idem | 5,0 kg/ha | 45 | 60 | 69 | 83 | 93 | 95 | 100 | 100 | 100 | 100 | 100 | 100 | 100 | 100 | 100 | 24 | 10 | 5 |
| Chlortoluron | 1,25 kg/ha | 0 | 33 | 8 | 60 | 82 | 88 | 100 | 100 | 100 | 98 | 100 | 100 | 100 | 100 | 100 | 0 | 10 | 0 |
| Chlortoluron | 2,5 kg/ha | 3 | 13 | 20 | 90 | 93 | 90 | 100 | 100 | 100 | 100 | 100 | 100 | 100 | 100 | 100 | 3 | 10 | 0 |

Exemple 14

Activité fongicide des composés (test d'inhibition de la croissance mycélienne).

Le milieu nutritif utilisé est le milieu Czapeck de composition suivante:

| | |
|---|---|
| Nitrate de soude | 2 g |
| Phosphate bipotassique | 1 g |
| Chlorure de potassium | 0,5 g |
| Sulfate de magnésie ($7H_2O$) | 0,5 g |
| Sulfate de fer ($7H_2O$) | 0,01 g |
| Saccharose | 30 g |
| Gélose | 15 g |
| Eau q sp | 1 000 ml |

Une suspension aqueuse du composé à tester est incorporée dans ce milieu maintenu en fusion à 45°C, à raison d'un ml de suspension aqueuse pour 9 ml de milieu Czapeck. La concentration en composé de la suspension aqueuse est telle que la concentration finale en composé dans le milieu nutritif est 100 ppm.

Le milieu est ensuite coulé dans des boîtes de Pétri de 90 mm de diamètre et on le laisse refroidir et solidifier. Chaque boîte est contaminée à l'aide de fragments de mycélium prélevés dans des cultures de champignons âgées de huit jours. Ces champignons sont füsarium roseum, rhizoctonia solani et alternaria tenuis.

On met les boîtes à incuber pendant 2 jours dans une chambre maintenue à 22°C et à un taux d'hygrométrie de 70%. Après ces 2 jours, le mycélium s'est développé réalisant autour du point de contamination des plages mycéliennes circulaires dont on mesure le diamètre.

Les résultats obtenus avec le composé de l'exemple 1 sont présentés dans le tableau IV. Dans ce tableau le pouvoir d'inhibition de la croissance mycélienne est traduit par une note (0,2 ou 4). La note 0 correspond à une inhibition nulle (dans ce cas le diamètre des plages mycéliennes dans les boîtes contenant le milieu nitritif traité avec le composé à tester est le même que le diamètre des plages mycéliennes dans les boîtes témoins contenant le milieu nutritif non traité). La note 2 correspond à une inhibition partielle, la note 4 à une inhibition totale. Dans le tableau IV sont donnés également les résultats obtenus avec un fongicide de référence (Thirame).

TABLEAU IV

| COMPOSE TESTE | CHAMPIGNON | | |
|---|---|---|---|
| | Fusarium roseum | Rhizoctonia solani | Alternaria tenuis |
| Exemple 1 | 2 | 2 | 2 |
| Thirame | 2 | 4 | 4 |

Exemple 15

Activité fongicide des composés (test d'inhibition de la germination des spores).

On utilise le même milieu nutritif que dans l'exemple 14 (milieu gélosé Czapeck) et on y incorpore le composé à tester de la même façon. La concentration finale en composé dans le milieu nutritif est 100 ppm.

Le milieu ainsi traité est coulé dans les alvéoles de plaques de ciréra et on laisse refroidir et solidifier. On contamine les lentilles de milieu nutritif en déposant sur chacune 50 $\mu l$ d'une suspension aqueuse de spores des espèces botrytis cinerea, alternaria tenuis, aspergilus niger, penicillium expansum. On met les plaques de ciréra dans des boîtes de Pétri de 15 cm de diamètre garnies au fond d'un papier filtre humide. On laisse incuber 24 heures à la température de 22°C. Après quoi, on compte, sous le microscope, le nombre de spores non germées et on compare ce nombre au nombre total de spores.

Les résultats obtenus avec les composés des exemples 1 et 11 et un fongicide de référence (Thirame) sont présentés dans le tableau V. Dans ce tableau le pouvoir d'inhibition de la germination des spores est exprimé suivant le système de notation indiqué à l'exemple 14 (note 0,2 ou 4).

TABLEAU V

| COMPOSE TESTE | CHAMPIGNON | | | |
|---|---|---|---|---|
| | Botrytis cinerea | Altemaria tenuis | Penicilium expansum | Aspergilus niger |
| Exemple 1 | 2 | 4 | 2 | 4 |
| Exemple 11 | 2 | 2 | 2 | 2 |
| Thirame | 4 | 4 | 4 | 4 |

Exemple 16

Activité fongicide des composés (test in vivo sur Erisiphe graminis, Phytophtora infestans et Uromyces Phaseoli).

Le composé à tester est formulé sous forme d'une suspension aqueuse dont la concentration en composé est 1 000 ppm. Cette suspension est pulvérisée sur les feuilles de plants d'orge "Rika" âgés de 10 jours ou de plants de tomate "Marmande" âgés de 20 jours ou de plants de haricot "Mange tout plein le panier" âgés de 12 jours, cultivés en pots. La quantité de suspension apportée par unité de surface des pots équivaut à 1 000 1/ha.

24 heures après le traitement, on saupoudre sur le feuillage des orges des spores d'Erisiphe graminis, on pulvérise sur le feuillage des tomates une suspension aqueuse de spores de Phytophtora infestans et on pulvérise sur le feuillage des haricots une suspension aqueuse de spores d'Uromyces phaseoli. On laisse ensuite les plantes en serre à 22°C.

6 jours après la contamination dans le cas des orges, 5 jours après la contamination dans le cas des tomates et 10 jours après la contamination dans le cas des haricots, on note le nombre de taches d'oïdium, de mildiou ou de rouille présentes sur les feuilles et on compare ce nombre à celui obtenu dans le cas des plantes témoins (c'est-à-dire des plantes non traitées avec le composé, mais contaminées). Les résultats sont exprimés suivant l'échelle de notation suivante:

note 0 : efficacité fongicide 0%
note 1 : efficacité fongicide 1 à 33%
note 2 : efficacité fongicide 34 à 66%
note 3 : efficacité fongicide 67 à 99%
note 4 : efficacité fongicide 100%

Les résultats obtenus avec les composés des exemples 1 et 5 et les fongicides de référence Manèbe et triadiméfon sont rassemblés dans le tableau VI ci-après.

TABLEAU VI

| COMPOSE TESTE | CHAMPIGNON | | |
|---|---|---|---|
| | Erisiphe graminis | Phytophtora infestans | Uromyces phaseoli |
| Exemple 5 | 4 | 3 | – |
| Exemple 1 | – | 3 | 3 |
| Triadiméton | 4 | – | 4 |
| Manèbe | – | 4 | – |

Exemple 17

Activité fongicide des composés (test in vivo sur Erisiphe graminis).

On opère comme à l'exemple 16, avec des suspensions aqueuses dont la concentration en composé est respectivement 1 000 ppm, 250 ppm, 62,5 ppm et 15,6 ppm. Les résultats obtenus, rassemblés dans le tableau VII ci-après, sont traduits par un chiffre qui représente le niveau de contamination des plantes traitées exprimé en pourcentage du niveau de contamination des plantes témoins. 0 signifie donc qu'aucune tache d'oïdium ne s'est développée, 100 que le niveau de contamination est identique à celui des plantes témoins.

TABLEAU VII

| CHAMPIGNON : ERISIPHE GRAMINIS | | |
|---|---|---|
| PRODUIT TESTE | CONCENTRATION EN PPM DE LA SUSPENSION AQUEUSE | % DE CONTAMINATION PAR RAPPORT AU TEMOIN |
| Composé de l'exemple 5 | 1 000 | 0 |
| idem | 250 | 0 |
| idem | 62,5 | 65 |
| idem | 15,6 | 100 |

Le composé de l'exemple 5, appliqué préventivement, exerce un effet fongicide sur l'oïdium de l'orge.

**Revendications**

1. Composés de formule:

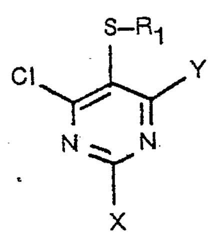

(I)

dans laquelle $R_1$ est un groupe alkyle ayant 1 à 5 atomes de carbone, l'un des substituants X et Y est un atome de chlore ou un groupe

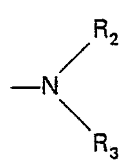

dans lequel $R_2$ est un atome d'hydrogène ou un groupe alkyle ayant 1 à 5 atomes de carbone et $R_3$ est un atome d'hydrogène ou un groupe alkyle ayant 1 à 5 atomes de carbone ou benzyle, et l'autre est un groupe azido, hydrazino, ou un groupe hydrazino substitué répondant à l'une des formules:

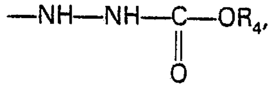

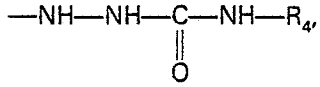

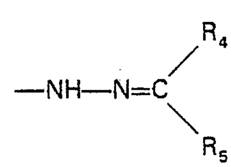

dans lesquelles $R_4$ et $R_5$ sont des groupes alkyle ayant 1 à 5 atomes de carbone, ou bien les substituants X et Y sont identiques et représentent alors des groups azido, hydrazino ou hydrazino substitués de formule

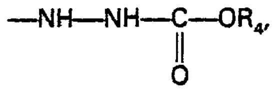, 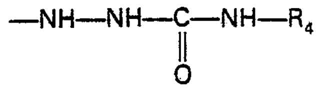 ou 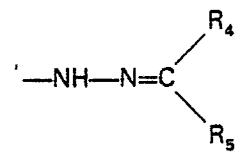

formules dans lesquelles $R_4$ et $R_5$ ont les significations précitées.

2. Composés selon la revendication 1 dans lesquels $R_1$ est un groupe méthyle, l'un des substituants X et Y est un atome de chlore ou un groupe amino, méthylamino et l'autre est un groupe azido, hydrazino,

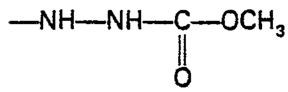 ou 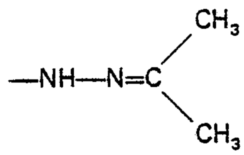 ,

ou bien les deux substituants X et Y sont des groupes hydrazino.

3. Mélanges de composés isomères de position répondant à la formule (I) de la revendication 1.

4. Procédé de préparation des composés de formule (I) de la revendication 1 dans lesquels l'un au moins des substituants X et Y est un groupe azido ou un groupe hydrazino substitué de formule

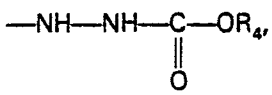, 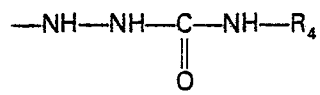 ou 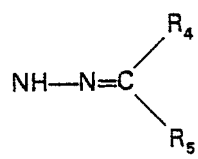

caractérisé en ce que l'on fait réagir le composé correspondant de formule (I) dans lequel l'un au moins des substituants X et Y est une groupe hydrazino avec respectivement l'acide nitreux, un chloroformiate de formule

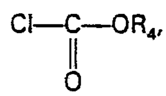,

un isocyanate de formule $R_4$—N=C=O, une cétone de formule

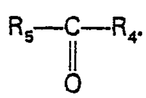.

5. Procédé de préparation des composés de formule (I) de la revendication 1 dans lesquels l'un des substituants X et Y est un atome de chlore ou un groupe

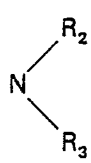

et l'autre est un groupe hydrazino caractérisé en ce que l'on condense une mole d'hydrazine avec une mole d'une trichloro-2,4,6 alkylthio-5 pyrimidine de formule:

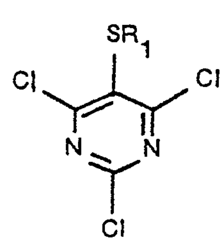

(II)

dans laquelle $R_1$ a la même signification que dans la revendication 1, et condense mole à mole la dichloro-4,6 (ou -2,6) hydrazino-2 (ou -4) alkylthio-5 pyrimidine ainsi obtenue avec un composé de formule

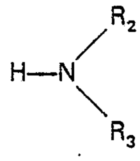

dans laquelle $R_2$ et $R_3$ ont les mêmes significations que dans la revendication 1.

6. Procédé de préparation des composés de formule (I) de la revendication 1 dans lesquels l'un des substituants X et Y est un groupe

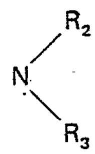

et l'autre est un groupe hydrazino, caractérisé en ce que l'on condense, mole à mole, l'hydrazine avec un composé de formule:

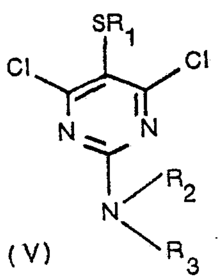

(V)

ou

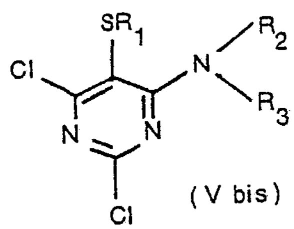

(V bis)

dans lesquelles $R_1$, $R_2$ et $R_3$ ont les mêmes significations que dans la revendication 1.

7. Procédé de préparation des composés de formule (I) de la revendication 1 dans lesquels X et Y sont tous les deux des groupes hydrazino, caractérisé en ce que l'on condense l'hydrazine avec une trichloro-2,4,6 alkylthio-5 pyrimidine de formule:

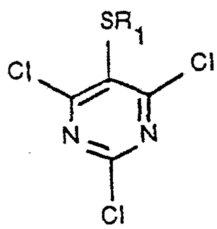

(II)

dans laquelle $R_1$ a la même signification que dans la revendication 1.

8. Application en tant que herbicides et fongicides des produits définis dans chacune des revendications 1 à 3.

9. Compositions herbicides contenant 1% à 95% en poids d'un produit tel que défini dans chacune des revendications 1 à 3, le complément à 100% étant constitué par des additifs inertes.

10. Compositions herbicides contenant 1% à 80% en poids d'un produit tel que défini dans chacune des revendications 1 à 3, 80% à 1% en poids d'un autre herbicide, le complément à 100% étant constitué par des additifs inertes.

**Patentansprüche**

1. Verbindungen der Formel

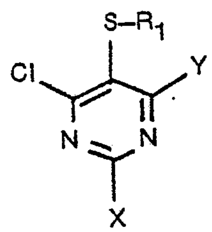

(I)

in der $R_1$ eine Alkylgruppe mit 1 bis 5 Kohlenstoffatomen, einer der Substituenten X und Y ein Chloratom oder eine Gruppe

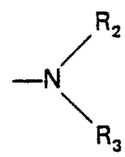

,

in der $R_2$ ein Wasserstoffatom oder eine Alkylgruppe mit 1 bis 5 Kohlenstoffatomen und $R_3$ ein Wasserstoffatom oder eine Alkylgruppe mit 1 bis 5 Kohlenstoffatomen oder Benzyl bedeuten, und der andere Substituent eine Azido-, Hydrazino- oder eine substituierte Hydrazinogruppe gemäß einer der folgenden Formeln bedeutet:

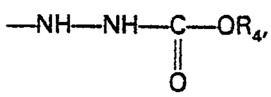 , 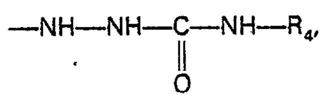 , 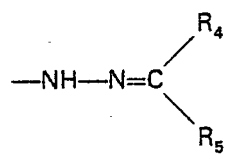

worin $R_4$ und $R_5$ Alkylgruppen mit 1 bis 5 Kohlenstoffatomen sind oder die Substituenten X und Y identisch sind und Azido-, Hydrazino- oder substituierte Hydrazinogruppen der Formeln:

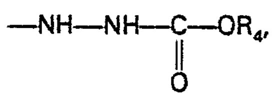 , 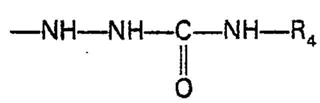 oder 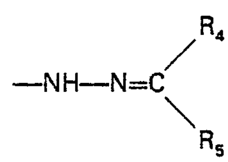

sind, in denen $R_4$ und $R_5$ die oben angegebenen Bedeutungen aufweisen.

2. Verbindungen nach Anspruch 1, in denen $R_1$ eine Methylgruppe, einer der Substituenten X und Y ein Chloratom, eine Amino- oder Methylaminogruppe und der andere eine Azido-, Hydrazino-,

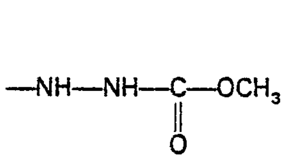 oder eine 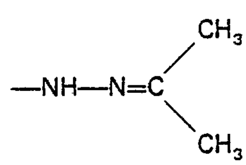

Gruppe bedeutet oder beide Substituenten X und Y Hydrazinogruppen sind.

3. Gemische aus stellungsisomeren Verbindungen gemäß Formel (I) des Anspruchs 1.

4. Verfahren zur Herstellung von Verbindungen der Formel (I) des Anspruchs 1, in denen mindestens einer der Substituenten X und Y eine Azido- oder substituierte Hydrazinogruppe der Formeln:

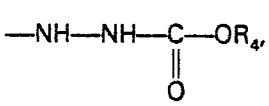 , 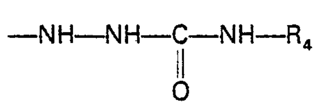 oder 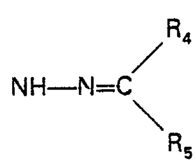

ist, dadurch gekennzeichnet, daß man die entsprechende Verbindung der Formel (I), in der mindestens

einer der Substituenten X und Y eine Hydrazinogruppe ist, mit salpetriger Säure, einem Chlorameisinsäureester der Formel

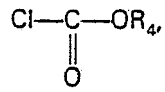

einem Isozyanat der Formel $R_4—N=C=O$, bzw. einem Keton der Formel

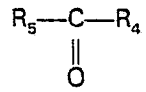

zur Umsetzung bringt.

5. Verfahren zur Herstellung von Verbindungen der Formel (I) des Anspruchs 1, in der einer der Substituenten X und Y eine Chloratom oder eine Gruppe

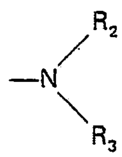

und der andere eine Hydrazinogruppe ist, dadurch gekennzeichnet, daß man 1 Mol Hydrazin mit 1 Mol 2,4,6-Trichlor-5-alkylthiopyrimidin der Formel

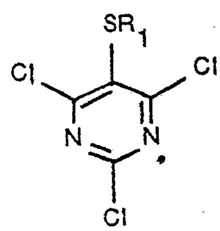

(II)

kondensiert, in der $R_1$ die gleiche Bedeutung wie in Anspruch 1 aufweist, und das auf diese Weise erhaltene 4,6-(oder 2,6-)-Dichlor-2(oder-4 )-hydrazino-5-alkylthiopyrimidin molweise mit einer Verbindung der Formel

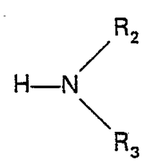

kondensiert, in der $R_2$ und $R_3$ die gleichen Bedeutungen wie in Anspruch 1 haben.

6. Verfahren zur Herstellung von Verbindungen der Formel (I) gemäß Anspruch 1, in denen einer der Substituenten X und Y eine

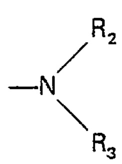

Gruppe und der andere eine Hydrazinogruppe ist, dadurch gekennzeichnet, daß man molweise Hydrazin mit einer Verbindung der Formel

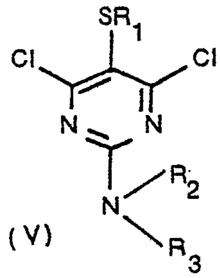

(V)

oder

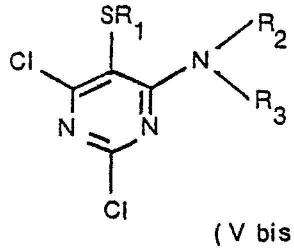

(V bis)

in denen $R_1$, $R_2$ und $R_3$ die gleichen Bedeugungen wie in Anspruch 1 haben, kondensiert.

7. Verfahren zur Herstellung von Verbindungen der Formel (I) gemäß Anspruch 1, in denen X und Y beide Hydrazinogruppen sind, dadurch gekennzeichnet, daß man Hydrazin mit einem 2,4,6-Trichlor-5-alkylthio-pyrimidin der Formel

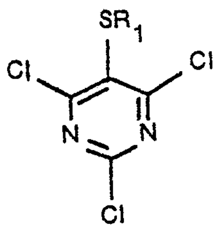

(II)

kondensiert, in der $R_1$ die gleiche Bedeutung wie in Anspruch 1 hat.

8. Verwendung der Verbindungen der Ansprüche 1 bis 3 als Herbizide und Fungizide.

9. Herbizide Zubereitung, enthaltend 1 bis 95 Gew.-% einer Verbindung gemäß den Ansprüchen 1 bis 3, wobei der Rest bis 100 Gew.-% aus inerten Additiven besteht.

10. Herbizide Zubereitung, enthaltend 1 bis 80 Gew.-% einer Verbindung gemäß einem der Ansprüche 1 bis 3 und 80 bis 1 Gew.-% eines anderen Herbizides, wobei der Rest bis 100 Gew.-% aus inerten Additiven besteht.

## Claims

1. Compounds of the formula:

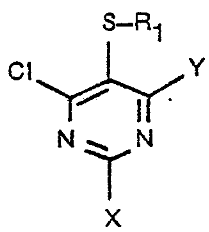

(I)

in which $R_1$ is an alkyl group having 1 to 5 carbon atoms, one of the substituents X and Y is a chlorine atom or an

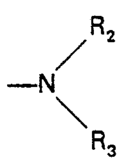

group in which $R_2$ is a hydrogen atom or an alkyl group having 1 to 5 carbon atoms, and $R_3$ is a hydrogen atom, or an alkyl group having 1 to 5 carbon atoms, or benzyl, and the other is an azido or hydrazino group, or a substituted hydrazino group corresponding to one of the formulae:

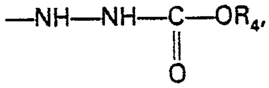,

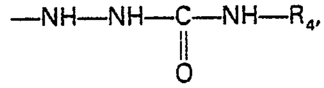,

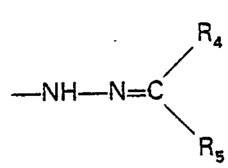

in which $R_4$ and $R_5$ are alkyl groups having 1 to 5 carbon atoms; or else the substituents X and Y are identical and thus represent azido, hydrazino or substituted hydrazino groups with the formula

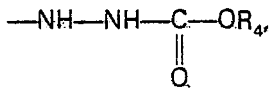

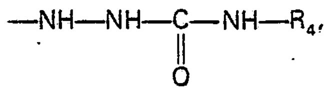

or

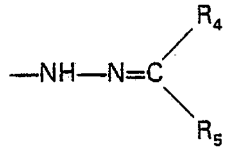

in which $R_4$ and $R_5$ have the significance given above.

2. Compounds according to claim 1 in which $R_1$ is a methyl group, one of the substituents X and Y is a chlorine atom or an amino or methylamino group and the other is an azido, hydrazino,

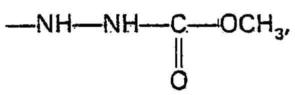

or

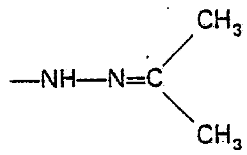

group or else the two substituents X and Y are hydrazino groups.

3. Mixtures of position isomeric compounds corresponding to the formula (I) of claim 1.

4. Process for the preparation of compounds of formula (I) of claim 1 in which at least one of the substituents X and Y is an azido group, or a substituted hydrazino group of formula

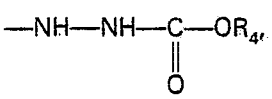

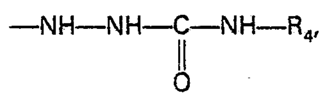

or

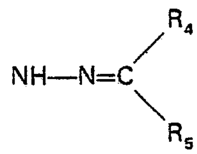

characterised in that the compound corresponding to formula (I) in which at least one of the substituents X and Y is a hydrazino group is reacted with, respectively, nitrous acid, a chloroformate of formula

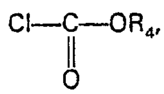

an isocyanate of formula $R_4—N=C=O$, a ketone of formula

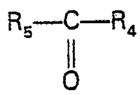

5. Process for the preparation of compounds of formula (I) of claim 1 in which one of the substituents X and Y is a chlorine atom or an

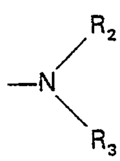

group and the other is a hydrazino group characterised in that one mole of hydrazine is condensed with one mole of a 2,4,6-trichloro-5-alkylthio pyrimidine of formula:

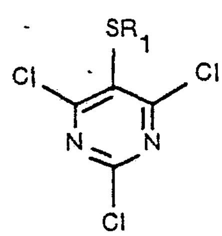

(II)

in which $R_1$ has the same significance as in claim 1 and the 4,6-(or 2,6-) dichloro-2-(or 4-) hydrazino-5-alkylthio pyrimidine thus obtained is condensed , mole for mole, with a compound of formula

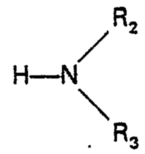

in which $R_2$ and $R_3$ have the same significance as in claim 1.

6. Process for the preparation of compounds of formula (I) of claim 1 in which one of the substituents X and Y is an

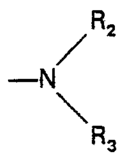

group, and the other is a hydrazino group, characterised in that hydrazine is condensed, mole for mole, with a compound of formula:

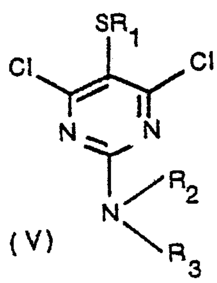

(V)

or

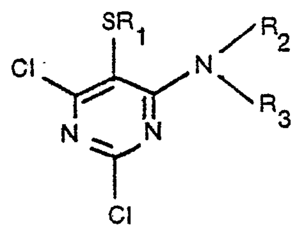

(V bis)

in which $R_1$, $R_2$ and $R_3$ have the same significance as in claim 1.

7. Process for the preparation of compounds of formula (I) of claim 1 in which X and Y are both hydrazino groups, characterised in that hydrazine is condensed with a 2,4,6-trichloro-5-alkylthio pyrimidine of formula:

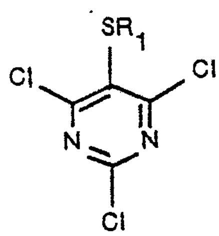

(II)

in which $R_1$ has the same significance as in claim 1.

8. Application as herbicides and fungicides of the products defined in each of claims 1 to 3.

9. Herbicidal compositions containing 1% to 95% by weight of a product such as that defined in each of claims 1 to 3, the complement to give 100% consisting of inert additives.

10. Herbicidal compositions containing 1% to 80% by weight of a product such as that defined in each of claims 1 to 3, 80% to 1% by weight of another herbicide, the complement to give 100% consisting of inert additives.